(19) Europäisches Patentamt
European Patent Office
Office européen des brevets

(11) EP 1 257 552 B1

(12) **FASCICULE DE BREVET EUROPEEN**

(45) Date de publication et mention de la délivrance du brevet:
**18.08.2004 Bulletin 2004/34**

(21) Numéro de dépôt: **01907784.1**

(22) Date de dépôt: **14.02.2001**

(51) Int Cl.[7]: **C07D 473/06**, C07D 473/04, A61K 31/522, A61P 19/10

(86) Numéro de dépôt international:
**PCT/FR2001/000420**

(87) Numéro de publication internationale:
**WO 2001/060824 (23.08.2001 Gazette 2001/34)**

# (54) DERIVES DE XANTHINE, INTERMEDIAIRES ET APPLICATION AU TRAITEMENT DE L'OSTEOPOROSE

XANTHINDERIVATE, ZWISCHENPRODUKTE UND VERWENDUNG ZUR BEHANDLUNG VON OSTEOPOROSIS

XANTHINE DERIVATIVES, INTERMEDIATES AND USE FOR TREATING OSTEOPOROSIS

(84) Etats contractants désignés:
**AT BE CH CY DE DK ES FI FR GB GR IE IT LI LU MC NL PT SE TR**

(30) Priorité: **15.02.2000 FR 0001845**

(43) Date de publication de la demande:
**20.11.2002 Bulletin 2002/47**

(73) Titulaire: **Aventis Pharma S.A.**
**92160 Antony (FR)**

(72) Inventeurs:
- **BILLEN, Günter, Johannes**
  **65527 Nierdernhausen (DE)**
- **BOUALI, Yamina**
  **F-94800 Villejuif (FR)**
- **NIQUE, François**
  **F-94170 Le Perreux sur Marne (FR)**
- **SATOH, Yusuke**
  **Kawagoe-shi, Saitama 350-1173 (JP)**
- **VEVERT, Jean-Paul**
  **F-93500 Pantin (FR)**

(74) Mandataire: **Rousseau, Pierrick Edouard**
**Aventis Pharma S.A.**
**Direction des Brevets,**
**Tri LEO/144**
**20, avenue Raymond Aron**
**92165 Antony Cedex (FR)**

(56) Documents cités:
**EP-A- 0 559 893**
**EP-A- 0 812 844**
**WO-A-96/36638**
**WO-A-98/35966**
**JP-A- 9 169 665**

- **CHEMICAL ABSTRACTS, vol. 130, no. 23, 7 juin 1999 (1999-06-07) Columbus, Ohio, US; abstract no. 306602, TAKAOKA K.: "Xanthine derivatives for prevention and treatment of bone diseases" XP002150857 & JP 11 092379 A (HOECHST MARION ROUSSEL K.K.) 6 avril 1999 (1999-04-06)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 257 552 B1

## Description

La présente invention a pour objet des dérivés de xanthine, leur procédé de préparation et les intermédiaires de ce procédé, leur application comme médicament et les compositions pharmaceutiques les renfermant.

L'ostéoporose est une pathologie diffuse du squelette caractérisée par une diminution de la masse osseuse (ostéopénie) à laquelle vient se rajouter une désorganisation de la microarchitecture de l'os conduisant à une diminution de la résistance mécanique de l'os et à une augmentation du risque fracturaire (ostéoporose).

L'ostéoporose est une maladie multifactorielle. C'est la ménopause, naturelle ou chirurgicale qui chez la femme constitue le facteur de risque primordial.

La perte osseuse post-ménopausique et l'ostéoporose, maladie, qui en en est la conséquence, résultent d'un déséquilibre du remodelage osseux au profit de la résorption osseuse par les ostéoclastes.

Cette ostéopénie est accélérée après la ménopause, du fait de la carence estrogénique, ce qui explique la large prédominance féminine de cette maladie.

Il en résulte une raréfaction progressive du tissu osseux, aboutissant à une fragilisation osseuse dont la conséquence clinique est la survenue de fractures spontanées.

L'estrogénothérapie est le traitement médicamenteux préventif de première intention des fractures ostéoporotiques, malgré les incertitudes concernant notamment les risques cancérologiques éventuels (sein et endomètre) ce qui limite son application.

Il convient donc de trouver des produits capables de prévenir la perte osseuse post ménopausique en cas de contre indication ou de « non désir » du traitement estrogénique.

Jusqu'à présent, seuls des inhibiteurs de la résorption osseuse (SERMs (Selective Estrogen Receptors Modulators)), les biphosphonates ou la calcitonine sont disponibles pour cette thérapeutique. Un des objectifs de la présente invention est par conséquence de mettre au point d'autres substances capables de stimuler la formation osseuse, d'augmenter réellement la masse osseuse et de restaurer la résistance mécanique.

L'invention a donc pour objet un dérivé de xanthine de formule (I) :

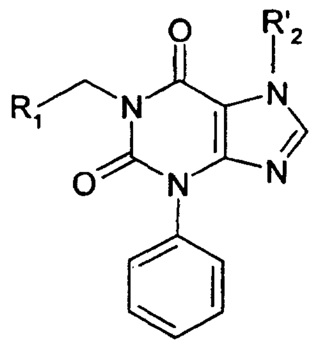

(I)

ainsi que leurs sels d'additions pharmaceutiquement acceptables dans laquelle

- $R'_2$ représente un atome d'hydrogène, un radical $(C_{1-6})$-alkyle ou aralkyle ou un groupement $-CH_2-O-(C_{1-6})$-alkyle, et

$R_1$ représente un hétérocycle choisi parmi :

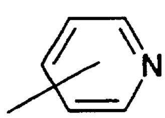

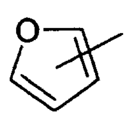

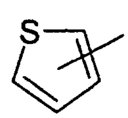

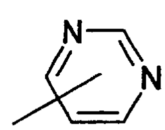

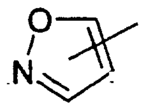 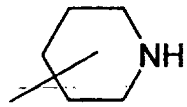 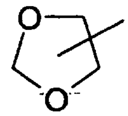 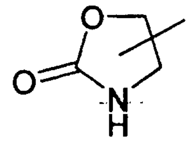

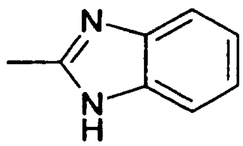 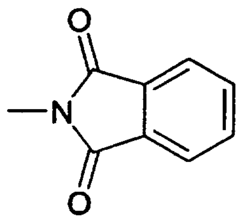 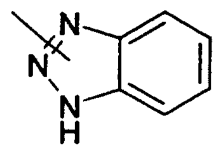

Parmi les composés préférés selon l'invention, on peut citer les composés de formules (I) dans lesquelles $R_1$ représente un groupement pyridin-4-yl.

Parmi les composés préférés de l'invention on peut citer le composé suivant :

- chlorhydrate de 3,7-dihydro-3-phényl-1-(4-pyridinylméthyl)-1H-purine-2,6-dione.

Lesdits composés de formule (I) peuvent être sous toutes leurs formes isomères possibles, seuls ou en mélange dans un rapport quelconque, et sous forme de promédicaments (prodrugs).

Par halogène on entend fluor, chlore, brome ou iode.

Les radicaux alkyles peuvent être linéaires ou ramifiés, saturés ou mono- ou poly-insaturés. Ceci s'applique également lorsqu'ils portent un substituant ou lorsqu'ils sont inclus dans des groupements tels que par exemple alkoxy, alkylamino, alkoxycarbonyle ou aralkyle.

Les atomes de carbone optiquement actifs contenus dans les composés de formule (I) peuvent indépendamment les uns des autres présenter la configuration R ou la configuration S.

Les composés de formule (I) peuvent être sous la forme d'énantiomères purs ou de diastéréoisomères purs ou sous la forme d'un mélange d'énantiomères, par exemple sous forme de racémates, ou de mélanges de diastéréoisomères.

La présente invention a donc pour objet les énantiomères purs, les mélanges de ces énantiomères, les diastéréoisomères purs et les mélanges de ces diastéréoisomères.

L'invention comprend les mélanges de deux ou plus de deux stéréoisomères de formule (I) et tous les rapports de ces stéréoisomères au sein des dits mélanges.

Les composés de formule (I) peuvent le cas échéant être présent sous forme d'isomères E ou d'isomères Z. L'invention a donc pour objet les isomères E purs, les isomères Z purs et les mélanges E/Z selon un rapport quelconque.

L'invention comprend également toutes les formes tautomères des composés de formule (I), par exemple en ce qui concerne la forme représentée par la formule (I), on considère également la forme dans laquelle, lorsque $R_2$ représente un atome d'hydrogène, la double liaison se trouve en position 5-6 de la xanthine au lieu de 4-5 et toutes les autres formes qui diffèrent par la position différente de l'atome d'hydrogène.

Enfin l'invention comprend les différents régioisomères notamment lorsque $R_1$ représente un hétérocycle.

Les diastéréoisomères, incluant les isomères E/Z peuvent être séparés en isomères individuels, par exemple par chromatographie. Les racémates peuvent être séparés en deux énantiomères par des méthodes courantes telles que la chromatographie en phase chirale ou par des méthodes de résolution.

Les sels physiologiquement acceptables des composés de formule (I) sont en particulier des sels pharmaceutiquement utilisables ou non toxiques ou physiologiquement utilisables.

Lorsque les composés de formule (I) renferment un groupe acide tel que l'acide carboxylique, il s'agit par exemple des sels de métaux alcalins ou alcaline terreux tels que les sels de sodium, de potassium, de magnésium, de calcium, et également les sels formés avec des ions ammonium quaternaires physiologiquement acceptables et les sels d'addition avec les acides tel que l'ammoniac et des amines organiques physiologiquement acceptables telles que par exemple la triéthylamine, l'éthanolamine ou le tris-(2-hydroxyéthyle)amine. Lorsque les composés de formule (I) contiennent un groupe basique, ils peuvent former un sel d'addition avec les acides par exemple avec les acides inorganiques tels que l'acide chlorhydrique, sulfurique, phosphorique ou avec les acides organiques carboxyliques

tels que l'acide acétique, trifluoroacétique, citrique, benzoïque, maléique, fumarique, tartrique, méthanesulfonique ou para toluène sulfonique.

Les composés de formule (I) qui comportent un groupe basique et un groupe acide, peuvent être présents sous forme de Zwiterions (bétaïnes), qui sont également inclus dans la présente invention.

Cet anion $Q^-$ physiologiquement acceptable qui est, le cas échéant, contenu dans les composés de formule (I), est de préférence un anion monovalent ou un équivalent d'anion polyvalent d'un acide organique ou inorganique non toxique, physiologiquement acceptable et pharmaceutiquement acceptable, par exemple l'anion ou un équivalent d'anion d'un des acides cités plus haut utile pour la formation des sels d'addition. $Q^-$ peut être par exemple un des anions (ou équivalent d'anion) d'un groupe choisi parmi chlorure, sulfate, phosphate, acétate, trifluoroacétate, citrate, benzoate, maléate, fumarate, tartrate, méthanesulfonate et para-toluènesulfonate.

Les sels des composés de formule (I) peuvent être obtenus par les méthodes ordinaires connues de l'homme du métier, par exemple en combinant un composé de formule (I) avec un acide organique ou inorganique ou une base dans un solvant ou un dispersant ou à partir d'un autre sel par échange de cation ou d'anion.

L'invention inclut également tous les sels des composés de formule (I) qui, à cause de leur faible acceptabilité physiologique, ne sont pas directement utilisable comme médicament, mais sont utilisables comme produits intermédiaires pour mettre en oeuvre des modifications chimiques ultérieures au niveau des composés de formule (I) ou comme produits de départ pour la préparation de sels physiologiquement acceptables.

La présente invention inclut également tous les solvates des composés de formule (I) par exemples les hydrates, les solvates formés avec les alcools, et tous les dérivés des composés de formule (I), par exemple les esters, prodrugs et autres dérivés physiologiquement acceptables, ainsi que les métabolites des composés de formule (I).

L'invention a plus particulièrement pour objet les prodrugs des composes de formule (I) qui peuvent être transformées en composés de formule (I) in vivo dans les conditions physiologiques. Les prodrugs des composés de formule (I), à savoir les dérivés chimiquement modifiés des composés de formule (I) afin d'obtenir des propriétés améliorées de manière désirée, sont connus de l'homme du métier.

Pour avoir plus d'information sur le type de prodrug envisagé dans la présente invention, on peut citer les ouvrages suivants : Fleicher et al., Advanced Drug Delivery Review 19 (1996) 115-130 ; Design of prodrugs, H. Bundgaard, Ed., Elsevier, 1985 ; H. Bungaard, Drugs of the Future 16 (1991) 443 ; Saulnier et al. Bioorg. Med. Chem. Lett. 4 (1994) 1985 ; Safadi et al. Pharmaceutical Res. 10 (1993) 1350. Parmi les prodrugs appropriées des composés de formule (I) on peut citer de préférence :

- les prodrugs sous forme d'esters des groupes carboxyliques, en particulier du groupe COOH, que peut représenter $R_5$.
- les prodrugs sous forme d'acyle et de carbamate pour les groupes contenant un azote acylable tel que les groupes amino. Dans les prodrugs acylés ou sous forme de carbamate, une ou plusieurs fois, par exemple deux fois, un atome d'hydrogène situé sur l'atome d'azote est remplacé par un groupe acyle ou carbamate. Parmi les groupes acyles ou carbamates préférés, on peut citer les groupes $R_{10}CO$-, $R_{11}OCO$-,

dans lesquels $R_{10}$ est un hydrogène ou un radical $(C_1\text{-}C_{18})$-alkyle, $(C_3\text{-}C_{14})$-cycloalkyle, $(C_3\text{-}C_{14})$-cycloalkyle-$(C_1\text{-}C_8)$-alkyle, $(C_5\text{-}C_{14})$-aryle, dans lequel 1 à 5 atomes de carbone peuvent être remplacés par des hétéroatomes tels que N, O, S ou $(C_5\text{-}C_{14})$-aryle-$(C_1\text{-}C_8)$alkyle, dans lequel 1 à 5 atomes de carbone dans la partie aryle peuvent être remplacés par des hétéroatomes tels que N, O, S et $R_{11}$ a les mêmes valeurs que $R_{10}$ à l'exception d'hydrogène.

La présente invention a également pour objet un procédé de préparation des composés de formule (I). Les composés peuvent généralement être préparés, par exemple au cours d'une synthèse convergente par couplage de deux ou plusieurs fragments qui peuvent être dérivés par rétrosynthèse des composés de formule (I). Afin d'éviter que les groupes fonctionnels puissent mener à des réactions indésirables ou secondaires au cours de chaque étape de synthèse, il peut être avantageux ou nécessaire au cours de la synthèse des composés de formule (I), d'introduire les groupes fonctionnels sous forme de précurseurs qui sont par la suite convertis en groupes fonctionnels désirés ou de bloquer temporairement ces groupes fonctionnels en mettant en oeuvre une stratégie de groupe protecteur appropriée à la synthèse qui est connue par l'homme de l'art (Greene, Wuts Protective Group in Organic Synthesis, Wiley 1991).

Ainsi les composés de formule (I) peuvent être préparés, par exemple, à partir d'une xanthine de formule (III) :

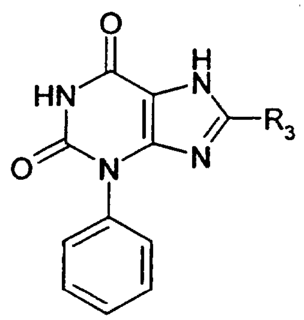

(III)

dans laquelle $R_3$ est telle que définie précédemment, qui subit l'une des réactions suivantes :

**soit**

a) protection de l'azote en position 7 afin d'obtenir le composé de formule (III) sous forme protégée

b) action d'un groupement $R_1$-$(CH_2)_n$-X en présence d'une base, R1 et n étant tels que définis précédemment et X étant un halogène ou un groupe partant, afin d'obtenir le composé intermédiaire de formule (IVa) :

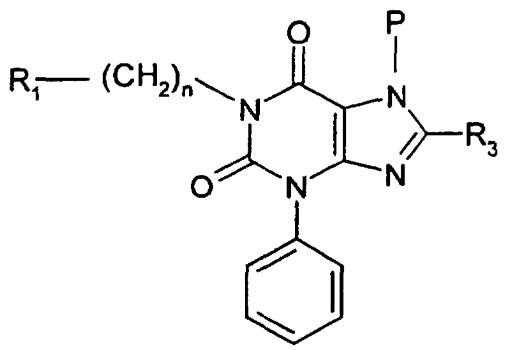

(IVa)

p étant un groupement protecteur,

c) déprotection de l'azote en position 7

d) le cas échéant action d'un groupement $R_2$-X en présence d'une base

**soit**

a) action d'un groupement $R_2$-X en présence d'une base afin d'obtenir le composé intermédiaire de formule (IVb)

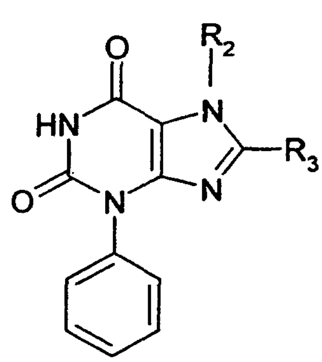

(IVb)

b) action d'un groupement $R_1$-$(CH_2)_n$-X en présence d'une base, R1 et n étant tels que définis précédemment et X étant un halogène ou un groupe partant, afin d'obtenir le composé formule (I)

Les réactions de protection, déprotection ainsi que les réactions de substitution nucléophiles mettant en oeuvre $R_2$-X ou $R_1$-$(CH_2)_n$-X sont connues de l'homme du métier. Le schéma ci-dessous illustre sans toutefois limiter le procédé selon l'invention, ainsi que la préparation du composé de formule (III).

La préparation des composés de formule (III) est également décrite dans Chem. Phar. Bull. 14(12) 1365-1370

(1966).

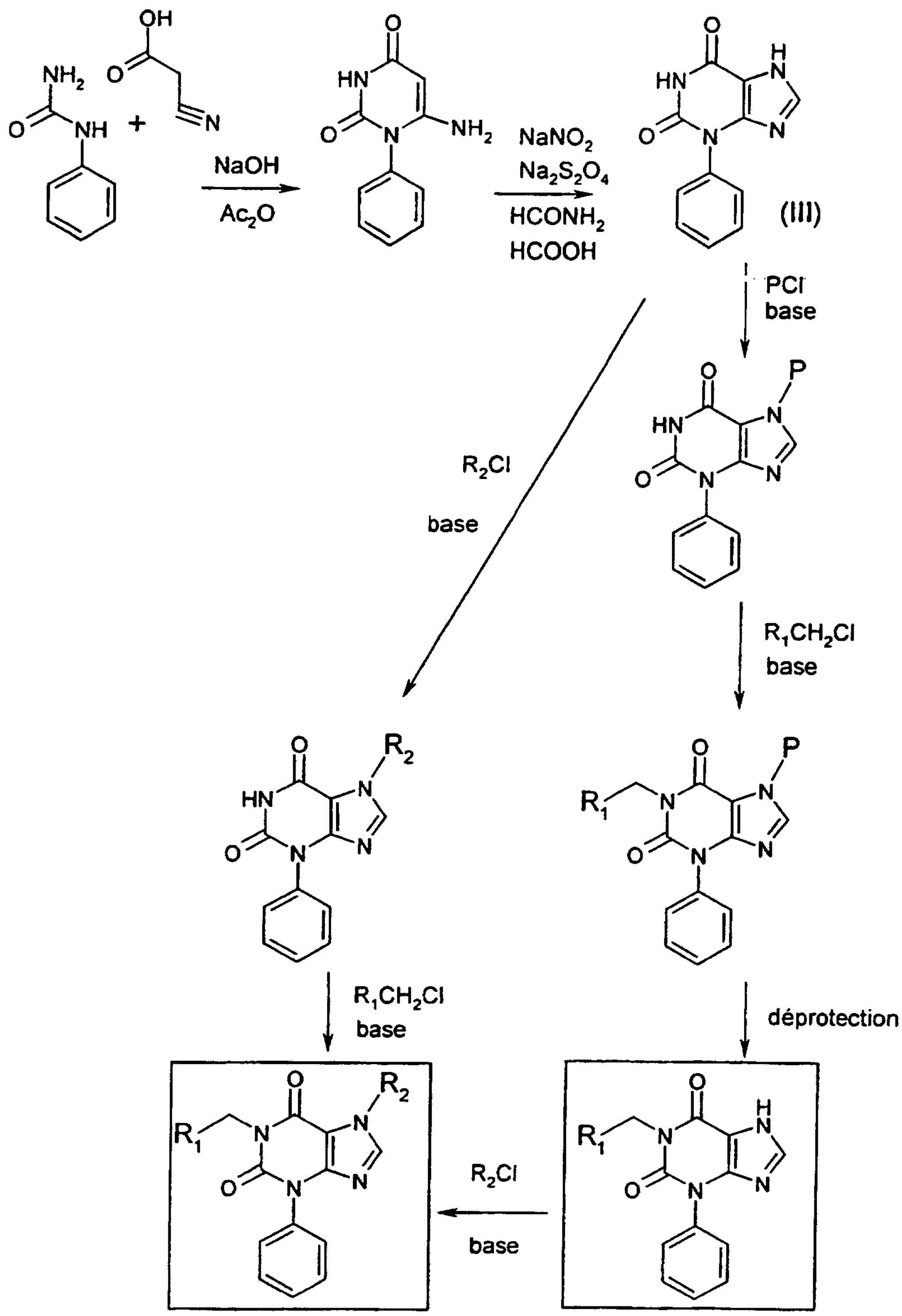


Les composés de formule (I) sont des composés ayant une activité pharmacologique et peuvent ainsi être utilisés à titre de médicaments dans le traitement ou la prévention des maladie de l'os.

Les composés de formule (I) ainsi que leurs sels physiologiquement acceptables et leurs prodrugs peuvent être administrés aux animaux, de préférence aux mammifères et en particulier aux êtres humains comme médicaments à titre thérapeutique ou prophylactique.

Ils peuvent être administrés tels quels ou en mélange avec un ou plusieurs autres composés de formule (I)

ou encore sous la forme d'une préparation pharmaceutique (composition pharmaceutique) qui permet une administration entérale ou parentérale et qui renferme à titre de composé actif une dose efficace d'au moins un composé de formule (I) et/ou ses sels physiologiquement acceptables et/ou ses prodrugs ainsi que des supports et/ou additifs courants et pharmaceutiquement inertes.

La présente invention a donc pour objet les composés de formule (I) et/ou leurs sels physiologiquement acceptables et/ou leurs prodrugs à titre de médicament.

La présente invention a également pour objet l'utilisation des composés de formule (I) et/ou leurs sels physiologiquement acceptables et/ou leurs prodrugs pour la préparation de médicaments destinés à la prévention ou au traitement des maladies citées plus haut ou plus bas, par exemple pour le traitement ou la prévention des maladies de l'os.

Les compositions pharmaceutiques qui permettent une administration entérale ou parentérale, renferment à titre de composé actif une dose efficace d'au moins un composé de formule (I) et/ou ses sels physiologiquement acceptables et/ou ses prodrugs ainsi qu'un ou plusieurs supports ou véhicule pharmaceutiquement inertes, et le cas échéant un ou plusieurs additifs usuels.

L'invention a donc pour objet une composition pharmaceutique renfermant au moins un médicament tel que défini précédemment ainsi qu'un ou plusieurs excipients.

Les médicaments peuvent être administrés oralement, par exemple sous forme de pilule, de comprimés, de comprimés enrobés, de pelliculés, de granules, de gélules et capsules molles, de solutions, de sirops, d'émulsion, de suspension ou de mélange d'aérosol.

L'administration peut cependant être effectuée par voie rectale, par exemple sous forme de suppositoire ou par voie parentérale, par exemple sous forme de solutions injectables ou d'infusions, de microcapsules ou d'implants, ou par voie percutanée, par exemple sous la forme de pommade, de solutions, de pigments ou de colorants, ou par d'autre voie telle que sous la forme d'aérosol ou de spray nasal.

Les compositions pharmaceutiques selon l'invention sont préparées selon des méthodes connues en soi, des supports organiques ou inorganiques, pharmaceutiquement inertes étant additionnés aux composés de formule (I) et/ou leurs sels physiologiquement acceptables et/ou leurs prodrugs. Pour la production de pilules, de comprimés, de comprimés enrobés et de capsules en gélatine dure, il est possible d'utiliser par exemple, du lactose, de l'amidon de maïs ou ses dérivés, du talc, de l'acide stéarique ou ses sels. Les supports convenables pour des capsules en gélatine molle ou pour les suppositoires sont par exemple les graisses, les cires les polyols semi-solides ou liquides, les huiles naturelles ou modifiées. Les véhicules appropriés pour la préparation de solutions, par exemple les solutions injectables, les émulsions ou les sirops sont par exemple l'eau, les alcools, le glycérol, les polyols, le sucrose, les sucres invertis, le glucose, les huiles végétales. Les supports convenables pour les microcapsules ou les implants sont par exemple les copolymères d'acide glyoxylique et d'acide lactique. Les préparations pharmaceutiques contiennent normalement de 0,5 % à 90 % en poids de composés de formule (I) et/ou leurs sels physiologiquement acceptables.

En plus des principes actifs et des supports, les préparations pharmaceutiques peuvent contenir des additifs tels que par exemple des diluants, des désintégrants, des liants, des lubrifiants, des agents mouillants, des stabilisants, des émulsifiants, des préservateurs, des agents sucrants, des colorants des agents de flaveurs ou des aromatisants, des épaississants, des agents tampons, et aussi des solvants ou des solubilisants ou des agents pour obtenir un effet retard et également des sels pour modifier la pression osmotique, des agents d'enrobage ou des antioxydants. Elles peuvent également contenir deux ou plusieurs composés de formule (I) et/ou leurs sels physiologiquement acceptables et/ou leurs prodrugs. En outre, en plus d'au moins un ou plusieurs composés de formule (I) et/ou leurs sels physiologiquement acceptables et/ou leurs prodrugs, ils peuvent contenir au moins un ou plusieurs autres principes actifs utilisables à titre thérapeutique ou prophylactique.

Les préparations pharmaceutiques (compositions pharmaceutiques) renferment normalement de 0,2 à 500 mg, et de préférence de 1 à 200 mg de composé de formule (I) et/ou leurs sels physiologiquement acceptables et/ou leurs prodrugs.

L'action des composés de formule (I) peut être démontrée par exemple dans un test dans lequel on évalue l'impact osseux du dérivé de xanthine sur un modèle d'ostéopénie induite sur une rate ovariectomisée.

Les maladies de l'os dont le traitement ou la prévention nécessite l'emploi des composés de formule (I), sont notamment l'ostéoporose post ménopausique, l'ostéoporose chez l'homme, l'hypercalcémie, l'ostéopénie, par exemple causée par les métastases osseuses, les désordres dentaires par exemple les parodontites, l'hyperparathyroïdisme, les érosions périarticulaires dans l'arthrite rhumatoïde, et la maladie de Paget. En outre les composés de formule (I) peuvent être utilisés pour soulager, empêcher ou traiter les désordres de l'os qui sont causés par les traitements, par les glucocorticoïdes, les thérapies liées à la prise de stéroïdes ou de corticostéroïdes (ostéoporose secondaire telles que l'ostéoporose cortisonique), les ostéoporoses liées à l'immobilisation ou par les déficiences d'hormones sexuelles mâles ou femelles.

Les composés de formule (I) peuvent également être utiles pour la reconstruction osseuse ou la consolidation

des fractures osseuses.

Tous ces désordres sont caractérisés par une perte osseuse, qui est basée par un défaut d'équilibre entre la formation osseuse et la destruction osseuse et qui peut être influencé favorablement par l'inhibition de la résorption osseuse par les ostéoclastes.

Lorsqu'on utilise les composés de formule (I), les doses peuvent varier à l'intérieur de limites larges et doivent être fixées en fonction de la personne à traiter. Ceci dépend par exemple du composé employé ou de la nature et de la sévérité de la maladie à traiter et si on se trouve dans des conditions graves ou chronique ou si on met en oeuvre un traitement prophylactique.

Dans le cas d'une administration par voie orale, la dose quotidienne varie en général de 0,01 à 100 mg/kg et de préférence de 0,1 à 50 mg/kg, en particulier de 0,1 à 5 mg/kg.

Dans le cas d'une administration par voie intraveineuse, la dose quotidienne varie approximativement de 0,01 à 100 mg/kg et de préférence de 0,05 à 10 mg/kg.

La dose quotidienne peut être divisée, en particulier dans le cas de l'administration de grande quantité de principe actif, en plusieurs, par exemple 2, 3 ou 4 parts. Le cas échéant, en fonction du comportement individuel, il peut être nécessaire d'administrer les différentes doses de manière croissante ou décroissante.

L'invention a encore pour objet, à titre de produits industriels nouveaux, les composés de formule ($IV_a$) ou ($IV_b$) tels que définis ci-dessus.

Les exemples suivants illustrent l'invention sans toutefois la limiter.

## Exemples :

Les produits ont été identifiés par spectre de masse (MS), infrarouge (IR) et/ou spectre RMN. Les composés, qui ont été purifiés par chromatographie en utilisant un éluant qui contient par exemple de l'acide acétique ou trifluoroacétique, et qui ensuite sont séchés ou dans lesquels lors de la dernière étape de synthèse, par exemple de l'acide trifluoroacétique a été utilisé pour éliminer un groupe protecteur tert-butyl, contiennent parfois, en fonction de la manière dont le produit a été séché, l'acide provenant de l'éluant ou de la dernière étape de synthèse et donc se trouvent partiellement ou complètement sous la forme du sel de l'acide utilisé, par exemple sous la forme d'un sel d'acide acétique ou trifluoroacétique. Ils peuvent également être plus ou moins hydratés.

Abréviations/noms chimiques : PCC : pyridine chlorochromate DMF : diméthylformamide ; THF : tétrahydrofurane ; MeOH : méthanol ; AcOEt : acétate d'éthyle ; TFA : acide trifluoroacétique ; TEA : triéthylamine ; $CH_2Cl_2$ : dichlorométhane ; ep. : (Épaulement) ; F : (fort) ; s : (singulet) ; d : (doublet) ; t : (triplet) ; 1 : (large) ; m : (multiplet).

### Exemple 1 : chlorhydrate de 3,7-dihydro-3-phényl-1-(4-pyridinylméthyl)-1H-purine-2,6-dione

Stade a : 3,7-dihydro-7-(éthoxyméthyl)-3-phényl-1H-purine-2,6-dione

A une solution de 3,7-dihydro-3-phényl-1H-purine-2,6-dione (12,11 g, 0,0536 mole) préparée selon Chem. Pharm. Bull. 14(12) 1365-1370 (1966) dans le DMF (120 ml) refroidie à 0-2°C, on ajoute l'hydrure de sodium (2,8 g, 2,2 eq), laisse remonter la température à environ 15 °C et ajoute le chlorométhyléthyléther (5,4 ml, 1,1 eq) et agite 4 heures à température ambiante. On évapore ensuite à pression réduite et purifie par chromatographie en éluant avec le mélange $CH_2Cl_2$/AcOEt 50/50. On obtient 6,51 g de produit attendu que l'on recristallise dans de l'éther au reflux puis 2 heures à température ambiante pour obtenir le produit purifié avec un rendement de 38,7 %.

SM

309Da = $MNa^+$

287Da = $MH^+$

RMN $CDCl_3$

1,24 (t), 3H, $CH_3$- ; 3,68 (q), 2H, C-$CH_2$-O ; 5,71 (s), 2H, N-$CH_2$-O ; 7,35 à 7,60 (m), 5H, $C_6H_5$ ; 7,72 (s), 1H, N-CH=N ; 8,60 (s), 1H, NH.

Stade b : 3,7-dihydro-7-(éthoxyméthyl)-3-phényl-1-(4-pyridinylméthyl)-1H-purine-2,6-dione

A une solution, sous argon et à 20°C, de xanthine préparée au stade précédent (4,8 g, 0,01676 mole) dans le DMF (48 ml), on ajoute le chlorure de 4-chlorométhylpyridinium (3,020 g, 1,1 eq) et du carbonate de potassium (5,56 g) et agite 1 h 30 à environ 70°C. On évapore ensuite sous pression réduite jusqu'à obtention d'un extrait sec que l'on purifie par chromatographie en éluant avec le mélange $CH_2Cl_2$/AcOEt 50/50. On obtient 5,78 g de produit attendu que l'on recristallise dans de l'éther diéthylique à 20°C pendant 1 heure. On obtient 5,37 g de produit attendu.

SM

$378^+$ = $MH^+$

$419^+ = MH^+ + CH_3CN$
$755^+ = 2MH^+$
RMN $CDCl_3$
1,24 (t), 3H, $CH_3$- ; 3,68 (q), 2H, C-$CH_2$-O ; 5,23 (s) , 2H, C-$CH_2$-N ; 5,73 (s), 2H, N-$CH_2$-O ; 7,72 (s), 1H, N-CH=N ; 7,41 à 7,55 (m), 5H, $C_6H_5$ ; 7,41 (d), 2H, H3 et H5 pyridinyl ; 8,55 (d), 2H, H2 et H6 pyridinyl.

Stade c : chlorhydrate de 3,7-dihydro-3-phényl-1-(4-pyridinylméthyl)-1H-purine-2,6-dione

A une solution de xanthine préparée au stade précédent (6,27 g, 0,0166 mole) dans 63 ml d'éthanol, on ajoute de l'acide chlorhydrique 6N (8,3 ml) et chauffe au reflux pendant 3 heures et 45 minutes. On évapore ensuite sous pression réduite jusqu'à obtention d'un extrait sec (6,69 g) que l'on recristallise dans de l'éther isopropylique (dissolution au reflux puis on ramène la température à 20°C et enfin agite 1 heure à 0-5°C). On obtient 5,69 g de produit attendu.
RMN DMSO $d_6$
5,37 (s), 2H, $CH_2$-N-CO ; 7,48 (m), 5H, $C_6H_5$ ; 8,02 (s), 1H, N-CH=N ; 8,07 (d), 2H, H3 et H5 pyridinyl ; 8,86 (d), 2H, H2 et H6 pyridinyl.

En opérant comme indiqué ci-desus, on a préparé les produits exemples 2 à 38 dont les noms suivent et les structures et analyses physiques figurent dans la tableau 1 ci-après.

Exemple 2 : 3,7-dihydro-1,7-bis(phénylméthyl)-3-phényl-1H-purine-2,6-dione

Exemple 3 : 3,7-dihydro-7-(éthoxyméthyl)-3-phényl-1-[[3-(trifluorométhyl)phényl]méthyl]-1H-purine-2,6-dione

Exemple 4 : 3,7-dihydro-3-phényl-1-[[3-(trifluoromethyl)-phényl]méthyl]-1H-purine-2,6-dione

Exemple 5 : 1-[(1H-benzotriazol-1-yl)méthyl]-3,7-dihydro-7-(éthoxyméthyl)-3-phényl-1H-purine-2,6-dione

Exemple 6 : 3,7-dihydro-7-(éthoxyméthyl)-3-phényl-1-[[4-(trifluorométhoxy)phényl]méthyl]-1H-purine-2,6-dione

Exemple 7 : 1-[(1H-benzotriazol-1-yl)méthyl]-3,7-dihydro-3-phényl-1H-purine-2,6-dione

Exemple 8 : 3,7-dihydro-7-(éthoxyméthyl)-3-phényl-1-(4-pyridinylméthyl)-1H-purine-2,6-dione

Exemple 9 : 3,7-dihydro-7-(éthoxyméthyl)-3-phényl-1-[[3,5-bis(trifluorométhyl)phényl]méthyl]-1H-purine-2,6-dione

Exemple 10 : 3,7-dihydro-7-(éthoxyméthyl)-1-[(2-fluoro-4-méthylphényl)méthyl]-3-phényl-1H-purine-2,6-dione

Exemple 11 : 3,7-dihydro-7-(éthoxyméthyl)-3-phényl-1-[[2-(phénylsulfonylméthyl)phényl]méthyl]-1H-purine-2,6-dione

Exemple 12 : 3,7-dihydro-7-(éthoxyméthyl)-3-phényl-1-[[3,4-bis(phénylméthoxy)phényl]méthyl]-1H-purine-2,6-dione

Exemple 13 : 3,7-dihydro-1-[[2,3-dihydro-2,3-dioxo-2H-isoindol-2-yl]méthyl]-7-(éthoxyméthyl)-3-phényl-1H-purine-2,6-dione

Exemple 14 : 3,7-dihydro-1-[(3,5-dimethyl-4-isoxazolyl)-méthyl]-7-(éthoxyméthyl)-3-phényl-1H-purine-2,6-dione

Exemple 15 : 3,7-dihydro-7-(éthoxyméthyl)-3-phényl-1-phénylméthyl-1H-purine-2,6-dione

Exemple 16 : 3,7-dihydro-3-phenyl-1-[[3,5-bis(trifluorométhyl)phényl]méthyl]-1H-purine-2,6-dione

Exemple 17 : 3,7-dihydro-1-[(2-fluoro-4-méthyl-phényl)-méthyl]-3-phényl-1H-purine-2,6-dione

Exemple 18 : 3,7-dihydro-1-[(2-méthyl-1-naphthalényl)méthyl]-3-phényl-1H-purine-2,6-dione

Exemple 19 : 3,7-dihydro-3-phényl-1-[[2-(phénylsulfonylméthyl)phényl]méthyl]-1H-purine-2,6-dione

Exemple 20 : 3,7-dihydro-3-phényl-1-[[3,4-bis(phénylméthoxy)-phényl]méthyl]-1H-purine-2,6-dione

Exemple 21 : 3,7-dihydro-1-[[2,3-dihydro-2,3-dioxo-2H-isoindol-2-yl]méthyl]-3-phényl-1H-purine-2,6-dione

Exemple 22 : 3,7-dihydro-1-[(3,5-diméthyl-4-isoxazolyl)-méthyl]-3-phényl-1H-purine-2,6-dione

Exemple 23 : 3,7-dihydro-3-phényl-1-phénylméthyl-1H-purine-2,6-dione

Exemple 24 : 3,7-dihydro-7-(éthoxyméthyl)-3-phényl-1-[(2-pyridinyl)méthyl]-1H-purine-2,6-dione

Exemple 25 : 3,7-dihydro-7-(éthoxyméthyl)-3-phényl-1-[(3-pyridinyl)méthyl]-1H-purine-2,6-dione

Exemple 26 : Chlorhydrate de 3,7-dihydro-3-phényl-1-[(2-pyridinyl)méthyl]-1H-purine-2,6-dione

Exemple 27 : Chlorhydrate de 3,7-dihydro-3-phényl-1-[(3-pyridinyl)méthyl]-1H-Purine-2,6-dione

Exemple 28 : 3,7-dihydro-3-phényl-1-[[(4-trifluorométhoxy)-phényl]méthyl]-1H-purine-2,6-dione

Exemple 29 : 3,7-dihydro-1-[(3,5-diméthyl-4-isoxazolyl)-méthyl]-3-phényl-7-propyl-1H-purine-2,6-dione

Exemple 30 : 3,7-dihydro-3-phényl-1-[(4-pyridinyl)méthyl]-7-propyl-1H-purine-2,6-dione

Exemple 31 : 3,7-dihydro-3-phényl-1-(phénylméthyl)-7-propyl-1H-purine-2,6-dione

Exemple 32 : 1-[(1H-benzotriazol-1-yl)méthyl]-3,7-dihydro-3-phényl-7-propyl-1H-purine-2,6-dione

Exemple 33 : 1-[(1H-benzotriazol-1-yl)méthyl]-3,7-dihydro-3-phényl-7-propyl-1H-purine-2,6-dione

Exemple 34 : 3,7-dihydro-1-[(3,5-diméthyl-4-isoxazolyl)-méthyl]-7-méthyl-3-phényl-1H-purine-2,6-dione

Exemple 35 : 3,7-dihydro-7-méthyl-3-phényl-1-[(4-pyridinyl)-méthyl]-1H-purine-2,6-dione

Exemple 36 : 3,7-dihydro-7-méthyl-3-phenyl-1-[(4-pyridinyl)-méthyl]-1H-purine-2,6-dione

Exemple 37 : 1-[(1H-benzotriazol-1-yl)méthyl]-3,7-dihydro-7-méthyl-3-phényl-1H-purine-2,6-dione

Exemple 38 : 3,7-dihydro-7-méthyl-3-phényl-1-[[(3-trifluorométhyl)phényl]méthyl]-1H-Purine-2,6-dione

**TABLEAU 1**

| Exemple | Structure | Analyse physique |
|---|---|---|
| 2 | 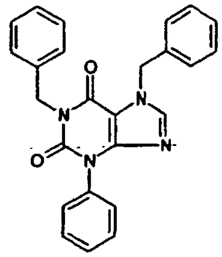 | RMN (DMSO)<br>5,09(sl) 2H et 5,52(sl) 2H : les $N-CH_2-ph$ ; de 7,18 à 7,54(m) 15H : les aromatiques ; 8,20(s) 1H : N=CH |
| 3 | 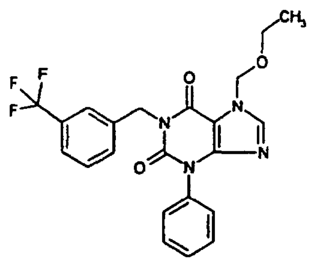 | F = 141°C |
| 4 | 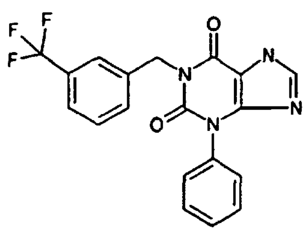 | F = 238°C |
| 5 | 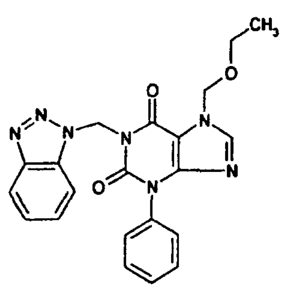 | RMN ($CDCl_3$)<br>1,24(t)3H : $\underline{CH_3}-CH_2-O-CH_2-N$<br>3,69(q)2H : $CH_3-\underline{CH_2}-O-CH_2-N$<br>5,75(s)2H : $CH_3-CH_2-O-\underline{CH_2}-N$<br>6,90(s) : $N-CH_2-N$<br>7,39(m)3H, 7,51(m)4H, 8,05(m) 2H : H aromatiques<br>7,72(s)1H : N=CH |

| Exemple | Structure | Analyse physique |
|---|---|---|
| 6 | 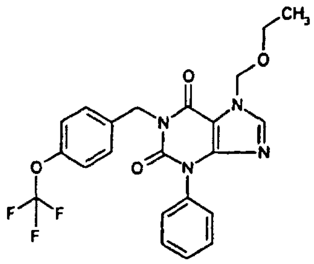 | RMN<br>1,24(t)3H : $CH_3$-$CH_2$-O-$CH_2$-N<br>3,67(q)2H : $CH_3$-$CH_2$-O-$CH_2$-N<br>5,23(s)2H : $CH_3$-$CH_2$-O-$CH_2$-N<br>5,74(s)2H : N-$CH_2$-ph<br>7,14-7,62=AB'BB'4H : O-ph<br>7,41(m)2H : les Ha<br>7,44 à 7,58(m)3H : les Hb+Hc |
| 7 | 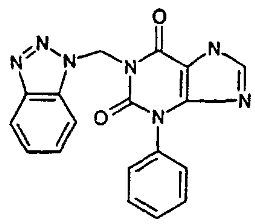 | F = 180°C |
| 8 | 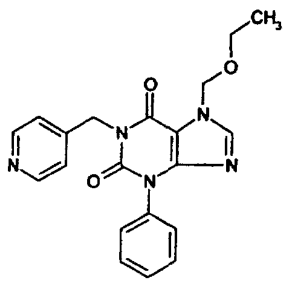 | F = 138°C |
| 9 | 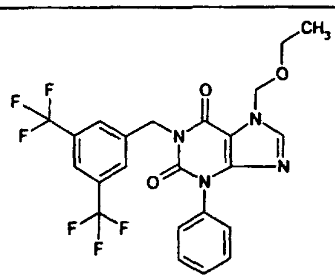 | F = 138°C |
| 10 | 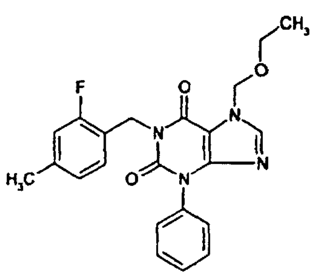 | F = 138°C |

| Exemple | Structure | Analyse physique |
| --- | --- | --- |
| 11 | 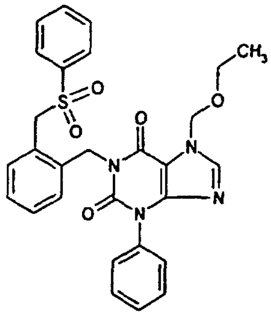 | F = 178°C |
| 12 | 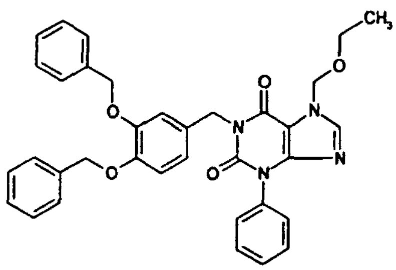 | RMN ($CDCl_3$)<br>1,23(t)3H : <u>CH</u>$_3$-$CH_2$-O-$CH_2$-N<br>3,66(q)2H : $CH_3$-<u>CH</u>$_2$-O-$CH_2$-N<br>5,73(s)2H : $CH_3$-$CH_2$-O-<u>CH</u>$_2$-N<br>5,13(m)6H : O-$CH_2$-ph + N-$CH_2$-ph<br>6,89(d)1H : Hc ; 7,11(dd)1H : Hb<br>7,27(d)1H : Ha<br>de 7,29 à 7,60(m)15H : phényles |
| 13 | 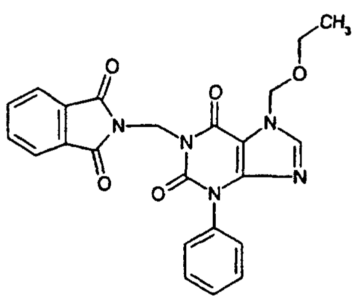 | RMN ($CDCl_3$)<br>1,24(t)3H : <u>CH</u>$_3$-$CH_2$-O-$CH_2$-N<br>3,71(q)2H : $CH_3$-<u>CH</u>$_2$-O-$CH_2$-N<br>5,76(s)2H : $CH_3$-$CH_2$-O-<u>CH</u>$_2$-N<br>6,06(s)2H : N-$CH_2$=N<br>7,71(m)-7,84(m) : phtalimide<br>7,38 à 7,57(m)5H : ph<br>7,71(s) : N=CH |
| 14 | 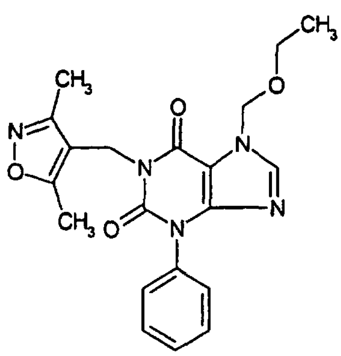 | F = 174°C |

| Exemple | Structure | Analyse physique |
|---|---|---|
| 15 | 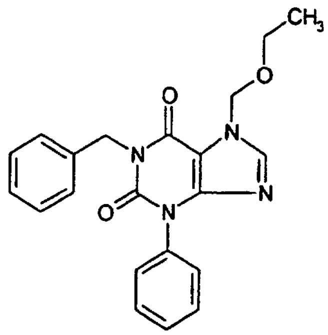 | RMN ($CDCl_3$)<br>1,24(t)3H : <u>$CH_3$</u>-$CH_2$-O-$CH_2$-N<br>3,67(q)2H : $CH_3$-<u>$CH_2$</u>-O-$CH_2$-N<br>5,74(s)2H : $CH_3$-$CH_2$-O-<u>$CH_2$</u>-N<br>5,25(s)2H : N-$CH_2$-ph<br>de 7,27 à 7,61(m)10H : les ph<br>7,68(s)1H : N=CH |
| 16 | 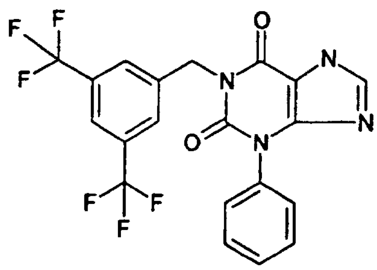 | RMN ($CDCl_3$)<br>5,39(s)2H : N-$CH_2$-ph;<br>7,44(m)2H : les $H_3$;<br>7,55(m)3H :$H_1$ et les $H_2$ ;<br>7,75(s)1H : N=CH ;<br>7,81(sl)1H : Hb ;<br>8,03(sl)2H : les Ha ;<br>12,09(sl)≤1H : NH |
| 17 | 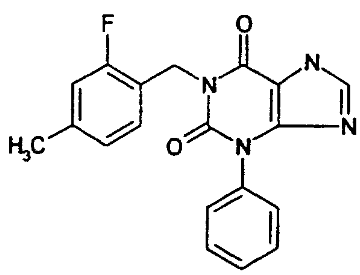 | F > 260°C |
| 18 | 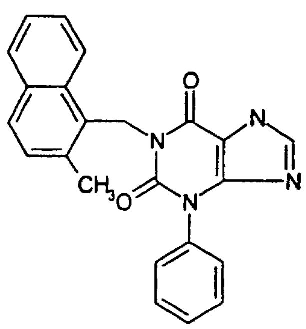 | F > 260°C |

| Exemple | Structure | Analyse physique |
|---|---|---|
| 19 | 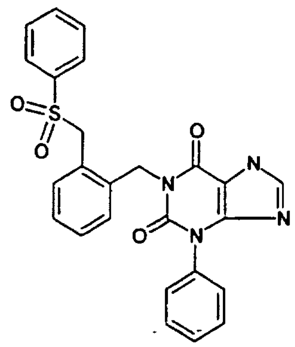 | RMN ($CDCl_3$)<br>4,84(s)2H : ph-$CH_2$-$SO_2$ ;<br>5,29(s)2H : ph-$CH_2$-N ;<br>6,89(dl)1H-7,13(td)1H-7,28(masqué)1H-7,61(m)1H : ph<<br>7,79(s)1H : N=CH ;<br>de 7,40 à 7,75(m)10H : -ph;<br>12,28(sl)1H : NH |
| 20 | 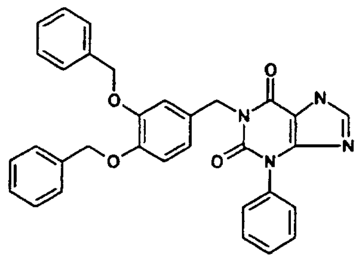 | RMN ($CDCl_3$)<br>5,11(sl)4H : les O-$CH_2$-ph;<br>5,19(sl)2H : les N-$CH_2$-ph ;<br>6,85(d)1H : Hc ;7,10(dd)1H : Hb;<br>de 7,18à 7,60(m)16H : Ha+les 3-ph ; 7,69(s)1H : N=CH ;<br>12,13(sl)1H : NH |
| 21 | 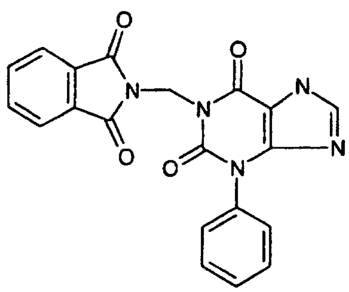 | F > 260°C |
| 22 | 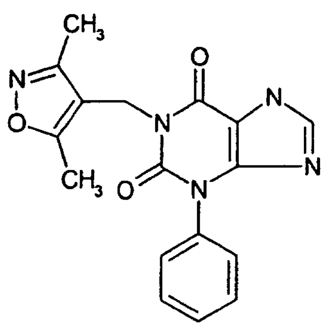 | F = 248°C |

| Exemple | Structure | Analyse physique |
|---|---|---|
| 23 | 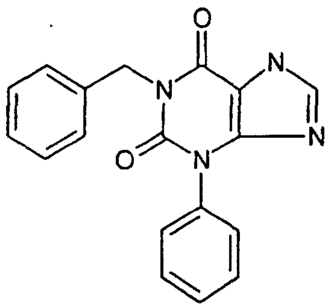 | F > 260°C |
| 24 | 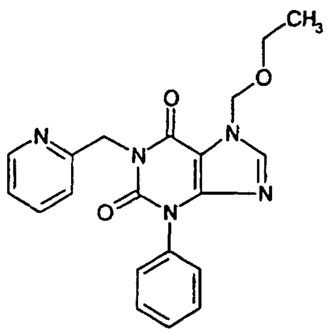 | RMN ($CDCl_3$)<br>1,23(t) : <u>$CH_3$</u>-$CH_2$-O-$CH_2$-N<br>3,69(q) : $CH_3$-<u>$CH_2$</u>-O-$CH_2$-N<br>5,75(sl)2H : $CH_3$-$CH_2$-O-<u>$CH_2$</u>-N<br>5,41(sl)2H : N-$CH_2$-C=<br>7,16(dd)1H : $H_5$ ; 7,31(d)1H :$H_3$ ;<br>7,64(td)1H : $H_4$ ; 8,53(dl)1H :$H_6$<br>de 7,39 à 7,57(m)5H :-ph ;<br>7,73(s) : N=CH |
| 25 | 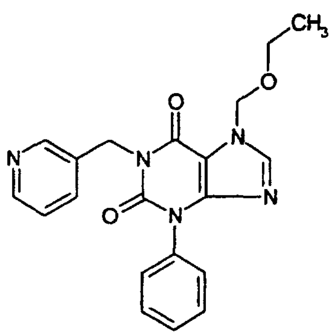 | RMN ($CDCl_3$)<br>1,24(t)3H : <u>$CH_3$</u>-$CH_2$-O-$CH_2$-N<br>3,68(q)2H : $CH_3$-<u>$CH_2$</u>-O-$CH_2$-N<br>5,74(sl)2H : $CH_3$-$CH_2$-O-<u>$CH_2$</u>-N<br>5,26(sl)2H : N-$CH_2$-C= ; 7,70(s)<br>1H : N=CH ; 7,40(m)2H : les Ha ;<br>de 7,44 à 7,59(m)3H : les<br>Hb+Hc ; 7,93 (ddd) : $H_4$ ;<br>8,53(dl) : $H_6$ ; 8,83(sl)1H : $H_2$<br>7,26 (masqué) : $H_5$ |
| 26 | 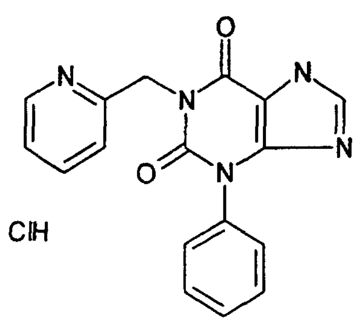 | RMN (DMSO)<br>5,40(sl)2H : N-$CH_2$-C=N<br>de 7,35 à 7,57(m)5H : -ph ;<br>8,71(dl)1H :$H_6$ ; 7,71(tl)1H :$H_5$<br>8,25(tl)1H : $H_4$ ; 7,80(dl)1H :$H_3$<br>8,02(s)1H : N=CH ; 5,21(sl) ≥<br>3H : H mobiles |

| Exemple | Structure | Analyse physique |
|---|---|---|
| 27 | 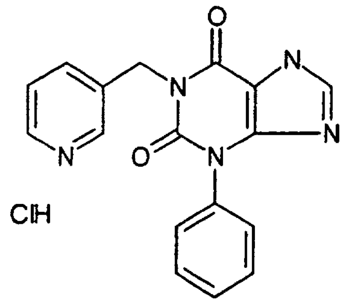 | RMN (DMSO)<br>5,28(sl)2H : $N-CH_2-C=$<br>de 7,41 à 7,56(m)5H : ph ;<br>7,98(dd)1H : $H_5$ ;<br>8,01(s)1H : N=CH ;<br>8,52(dl)1H : $H_4$ ;<br>8,91(dl)1H : $H_6$ ;<br>8,93(sl)1H : $H_2$ |
| 28 | 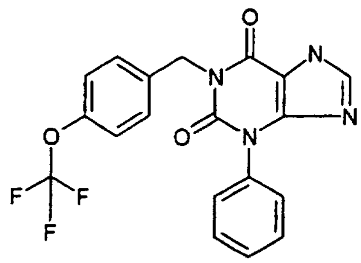 | F = 203°C |
| 29 | 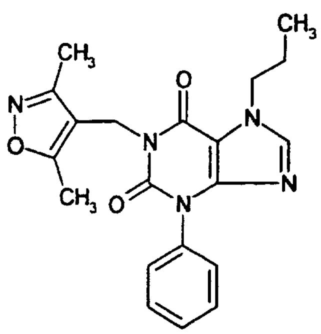 | F = 186°C |
| 30 | 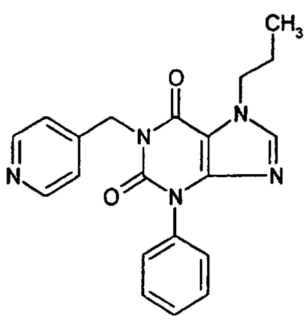 | F = 200°C |

| Exemple | Structure | Analyse physique |
|---|---|---|
| 31 | 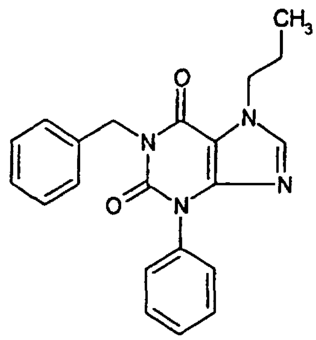 | F = 173°C |
| 32 | 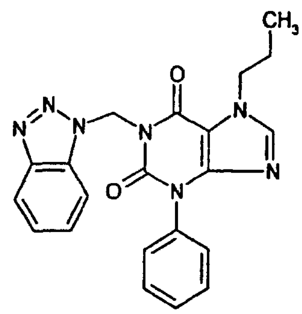 | RMN ($CDCl_3$)<br>0,98(t) : $\underline{CH_3}$-$CH_2$-$CH_2$-N<<br>1,96(m) : $CH_3$-$\underline{CH_2}$-$CH_2$-N<<br>4,30(t) : $CH_3$-$CH_2$-$CH_2$-N<<br>6,91(s) : N-$CH_2$-N<<br>7,50(s),8,06(d),7,37(m),7,50(m), 8,05(d) : benzotriazole ;<br>de 7,35 à 7,85(m) : les aromatiques |
| 33 | 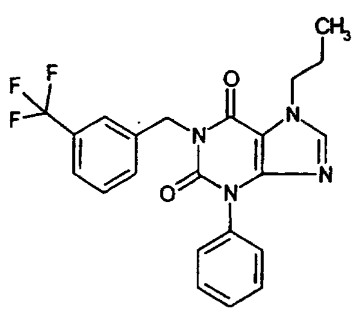 | F = 125°C |
| 34 | 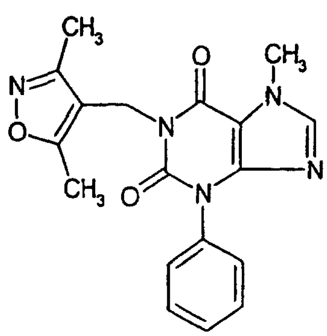 | F = 248°C |

| Exemple | Structure | Analyse physique |
|---|---|---|
| 35 | 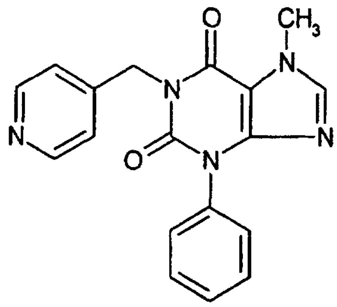 | F = 243°C |
| 36 | 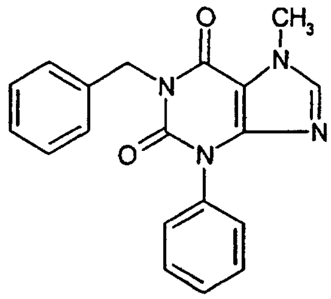 | F = 206°C |
| 37 | 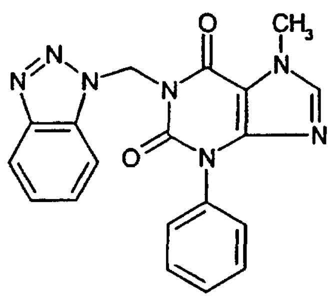 | F > 260°C |
| 38 | 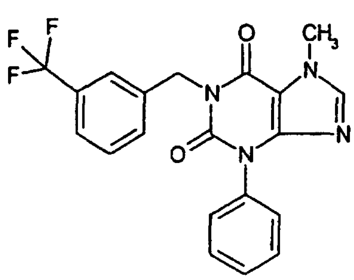 | F = 186°C |

## Tests pharmacologiques

L'impact osseux de ces dérivés xanthine a été évalué sur un modèle d'ostéopénie, la rate ovariectomisée à l'âge de 3 mois, afin de déterminer ses effets sur la masse osseuse, et sur l'activité de formation et de résorption osseuse. Les animaux sont traités en préventif.

**Animaux :**

| | |
|---|---|
| Espèce | rat |
| Souche | Sprague-Dawley |
| Sexe | femelle |
| Poids | 250 g à 300 g |
| Nbre d'animaux/groupe | 8 |

**Produits :**

Sur ce modèle d'ostéopénie l'effet de ces dérivés xanthine est comparé à celui de la PTH

- Produit à tester : composé de l'exemple 1

  - véhicule(s) : méthylcellulose 0,5 % ou sérum physiologique
  - dose(s) : 10 mg/kg pour le produit.

  la PTH est administrée à la dose de 10 µg ou 25 µg/kg/j par voie sous cutanée

  - nombre d'administrations : une fois/jour ; 5 jours/semaine pendant 4 semaines
  - voie d'administration : voie orale pour le produit. La PTH est administrée par voie sous cutanée
  - volume : 1 ml/kg p o et s c
  - délai entre l'injection et le sacrifice : 24 heures
  - nombre d'administrations : 20

**Chirurgie :**

Des rats femelles âgées de 3 mois et pesant environ 250-300 g sont ovariectomisées sous anesthésie à l'Imalgène 1000, à la dose de 100 mg/kg par voie intrapéritonéale (i.p) et sous un volume de 1 ml/kg. Ils reçoivent également du Nembutal (3 mg/kg i.p. sous un volume de 0,3 ml/kg). Les animaux sont répartis en groupes de 8 rats. Après incision latérale, les plans cutanés et musculaires sont sectionnés. L'exérèse de chaque ovaire se fait après ligature de l'oviducte. Les rats "SHAM" sont anesthésiés dans les mêmes conditions. Après incision des plans cutanés et musculaires, chaque ovaire est exposé puis replacé in situ.

**Traitements**

Les effets des produits sont déterminés après un traitement préventif. Les traitements débutent 24 heures après ovariectomie. Les animaux sont traités pendant 4 semaines, et se répartissent de la façon suivante :

- un groupe témoin Sham recevant le ou les véhicules
- un groupe témoin OVX recevant le ou les véhicules
- OVX + dérivé xanthine
- OVX + PTH

Prélèvements sanguins

Au terme des 4 semaines de traitement, les animaux sont décapités par guillotine. Les sérums recueillis après centrifugation sont conservés à -20°C.
Un bilan lipidique sera établi à partir des dosages sériques du cholestérol total, des triglycérides et des phospholipides

sur une aliquote de sérum de 500 µl.
La baisse du taux de cholestérol sérique est exprimée en % par rapport au taux présenté par les animaux ovariectomisés ne recevant que le solvant.

Prélèvements d'organes

Après sacrifice des animaux, les organes suivants sont prélevés :

- tractus génital

Les utérus sont prélevés. Ces derniers sont pesés. L'augmentation du poids est exprimée, en % du poids de l'utérus des animaux ovariectomisés ne recevant que le solvant.

- au niveau osseux :

La masse osseuse (BMD ou Bone Mineral Density = densité minérale osseuse) est mesurée par absorptiométrie biphotonique à rayons X à double énergie (DEXA). Les mesures sont réalisées sur les os excisés et débarrassés de tous les tissus mous. La BMD (Bone Minéral Density ) est mesurée sur l'os entier ainsi que sur la partie métaphysaire au niveau de l'extrémité proximale pour le tibia gauche. Cette zone est définie comme étant la région la plus riche en os trabéculaire ; et par conséquent, est la plus sensible aux variations de volume osseux et de densité minérale osseuse.
Les résultats sont exprimés en % selon la formule :

$$\frac{\text{BMD traitement testé - BMD OVX}}{\text{BMD SHAM -BMD OVX}} \times 100$$

| | Dose | OS TIBIA | UTERUS | Cholestérol |
|---|---|---|---|---|
| | Mg/kg | Densité % | Poids % | % |
| ex 1 | 10 mg/kg po | 60 % | 15% | Pas d'effet |
| PTH | 25 µg/kg sc | 100% | 9% | Pas d'effet |
| PTH | 10 µg/kg sc | 69% | Pas d'effet | Pas d'effet |
| OVX | Véhicule(s) | 0% | | |
| SHAM | Véhicule(s) | 100% | | |

Conclusion :

Utilisé à la dose de 10 mg/kg sur le modèle de la rate ovariectomisée ce produit offre une protection osseuse efficace (60 %), comparable à celle induite par la PTH (69 %) à la dose de 10 µg/kg alors que 25 µg/kg de PTH offrent 100 % de protection osseuse. Le composé ne présente en outre aucune activité utérotrophique, et pas d'effet sur le taux sérique de cholestérol.
Ce produit peut être utilisé comme médicament dans la prévention ou le traitement des ostéoporoses.

**Revendications**

1. Un dérivé de xanthine de formule (I)

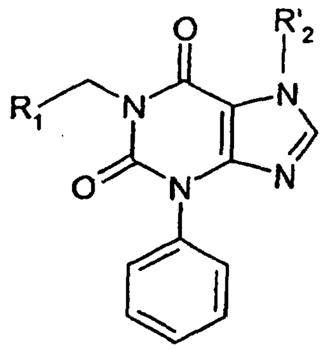

(I)

ainsi que ses sels d'additions pharmaceutiquement acceptables dans laquelle :

R'$_2$ représente un atome d'hydrogène, un radical ($C_{1-6}$)-alkyle ou aralkyle ou un groupement -$CH_2$-O-($C_{1-6}$)-alkyle.
$R_1$ représente un hétérocycle choisi parmi :

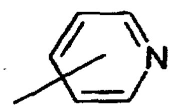 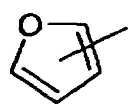 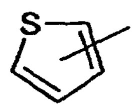 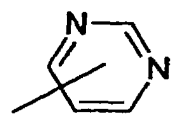

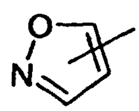 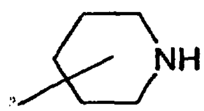 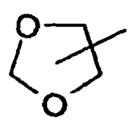 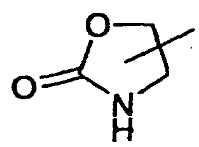

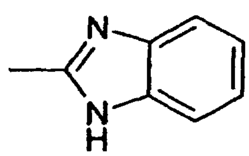 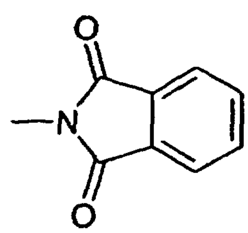 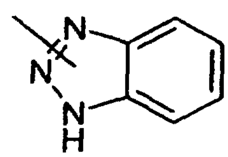

**2.** Un dérivé de xanthine de formule (I) telle que définie à la revendication 1 dans laquelle $R_1$ représente un groupement pyridin-4-yl.

**3.** Dérivé de xanthine de formule (I) telle que définie à l'une quelconque des revendications 1 à 2 dont le nom suit :
- chlorhydrate de 3,7-dihydro-3-phényl-1-(4-pyridinylméthyl)-1H-purine-2,6-dione.

**4.** Procédé de préparation des composés de formule (I) telle que définie à la revendication 1 au cours d'une synthèse convergente par couplage de deux ou plusieurs fragments qui peuvent être dérivés par rétrosynthèse des composés de formule (I).

**5.** Procédé, selon la revendication 4, de préparation des composés de formule (I) telle que définie à la revendication

1 **caractérisé en ce qu'**une xanthine de formule (III) :

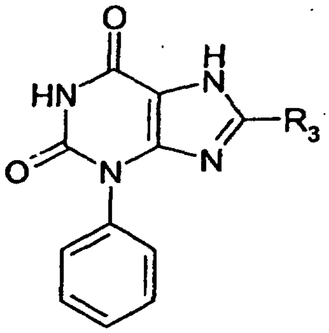

(III)

dans laquelle $R_3$ est telle que définie à la revendication 1, subit l'une des réactions suivantes :

**soit**

a) protection de l'azote en position 7 afin d'obtenir le composé de formule (III) sous forme protégée,
b) action d'un groupement $R_1$-$(CH_2)_n$-X en présence d'une base, $R_1$ est tel que défini précédemment et X étant un halogène ou un groupe partant, afin d'obtenir le composé intermédiaire de formule (IVa) :

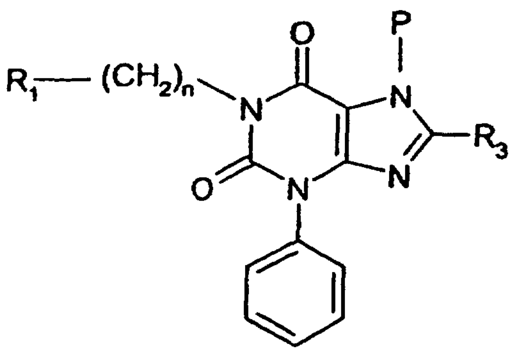

(IVa)

P étant un groupement protecteur,
c) déprotection de l'azote en position 7,
d) le cas échéant action d'un groupement $R_2$-X en présence d'une base,

**soit**

a) action d'un groupement $R_2$-X en présence d'une base afin d'obtenir le composé intermédiaire de formule (IVb) :

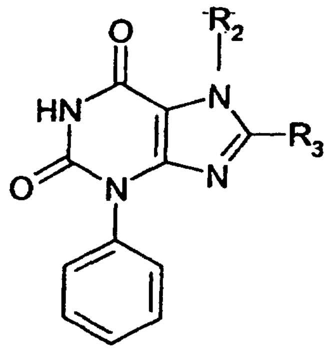

(IVb)

b) action d'un groupement $R_1$-$(CH_2)_n$-X en présence d'une base, $R_1$ et n étant tels que définis précédemment et X étant un halogène ou un groupe partant, afin d'obtenir le composé formule (I).

**6.** Composés de formule (I) tels que définis à l'une quelconque des revendications 1 à 2 et/ou leurs sels pharmaceutiquement acceptables et /ou leurs prodrugs à titre de médicament.

**7.** Composé tel que défini à la revendication 3 à titre de médicaments.

**8.** Médicaments tels que définis à la revendication 6 ou 7 destiné au traitement ou à la prévention de l'ostéoporose.

**9.** Compositions pharmaceutiques qui renferment à titre de composé actif, un médicament tel que défini aux revendications 6 à 8 et un ou plusieurs excipients.

**10.** A titre de produits industriels, les composés de formule (IVa) tels que définis à la revendication 5.

**Patentansprüche**

**1.** Xanthinderivat der Formel (I)

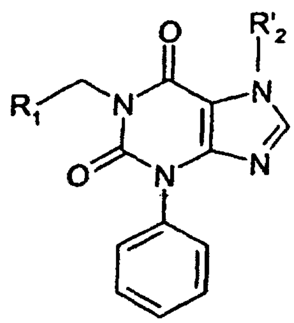

(I)

und seine pharmazeutisch unbedenklichen Additionssalze, worin
$R'_2$ ein Wasserstoffatom, einen $(C_{1-6})$-Alkylrest oder einen Aralkylrest oder eine $-CH_2-O-(C_{1-6})$-Alkylgruppe bedeutet und
$R_1$ einen Heterocyclus aus der Gruppe

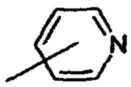 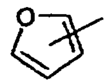 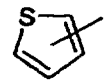 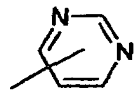

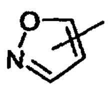 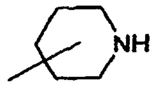 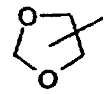 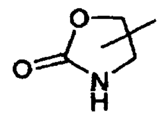

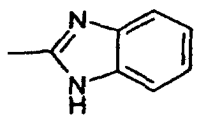 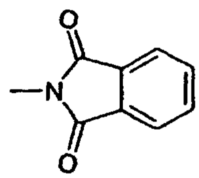 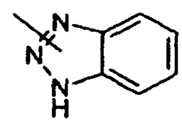

bedeutet.

**2.** Xanthinderivat der Formel (I) nach Anspruch 1, in der $R_1$ eine Pyridin-4-yl-Gruppe bedeutet.

**3.** Xanthinderivat der Formel (I) nach Anspruch 1 oder 2, mit der Bezeichnung 3,7-Dihydro-3-phenyl-1-(4-pyridinylmethyl)-1H-purin-2,6-dion-Hydrochlorid.

**4.** Verfahren zur Herstellung von Verbindungen der Formel (I) nach Anspruch 1 bei einer konvergenten Synthese mittels Kopplung von zwei oder mehreren Fragmenten, die durch Rücksynthese der Verbindungen der Formel (I) erhalten werden können.

**5.** Verfahren nach Anspruch 4 zur Herstellung von Verbindungen der Formel (I) nach Anspruch 1, **dadurch gekennzeichnet, daß** mit einem Xanthin der Formel (III),

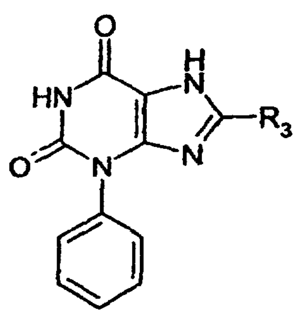

(III)

in der $R_3$ wie in Anspruch 1 definiert ist, eine der folgenden Reaktionen durchgeführt wird:

**entweder**

a) Schützen des Stickstoffs in 7-Stellung, um zu der Verbindung der Formel (III) in geschützter Form zu gelangen,
b) Einwirkenlassen einer $R_1$-$(CH_2)_n$-X-Gruppe in Gegenwart einer Base, wobei $R_1$ wie in Anspruch 1

definiert ist und n ist 1 und X ein Halogen oder eine Abgangsgruppe bedeutet, wodurch man zum Zwischenprodukt der Formel (IVa),

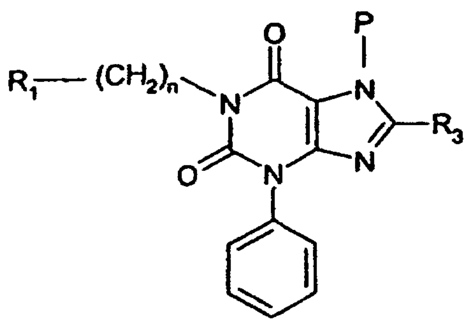

(IVa)

wobei P eine Schutzgruppe bedeutet,
gelangt,
c) Entschützen des Stickstoffs in 7-Stellung,
d) gegebenenfalls Einwirkenlassen einer $R_2$-X-Gruppe in Gegenwart einer Base,

**oder**

a) Einwirkenlassen einer $R_2$-X -Gruppe in Gegenwart einer Base, wodurch man zu dem Zwischenprodukt der Formel (IVb)

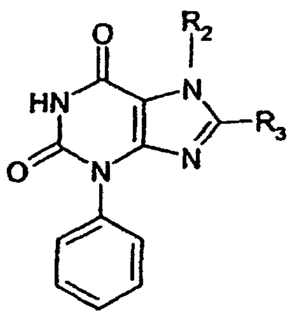

(IVb)

gelangt,
b) Einwirkenlassen einer $R_1$- $(GH_2)_n$-X-Gruppe in Gegenwart einer Base, wobei $R_1$ und n wie oben definiert sind und X ein Halogen oder eine Abgangsgruppe bedeutet, wodurch man zu der Verbindung der Formel (I) gelangt.

**6.** Verbindungen der Formel (I) nach Anspruch 1 oder 2 und/oder ihre pharmazeutisch unbedenklichen Salze und/oder ihre Prodrugs als Arzneimittel.

**7.** Verbindungen nach Anspruch 3 als Arzneimittel.

**8.** Arzneimittel nach Anspruch 6 oder 7 für die Behandlung oder Vorbeugung von Osteoporose.

**9.** Pharmazeutische Zusammensetzungen, die als Wirkstoff ein Arzneimittel nach den Ansprüchen 6 bis 8 sowie einen oder mehrere Grundstoffe enthalten.

**10.** Verbindungen der Formel (IVa) nach Anspruch 5 als Industriestoffe.

## Claims

1. Xanthin derivative of the formula (I)

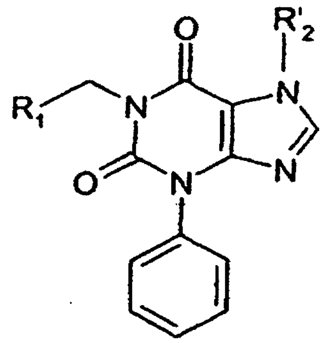

(I)

and its pharmaceutically acceptable addition salts, in which
R'$_2$ represents a hydrogen atom, a $(C_{1-6})$-alkyl or aralkyl radical or a $-CH_2-O-(C_{1-6})$-alkyl group, and
$R_1$ represents a heterocycle selected from amongst:

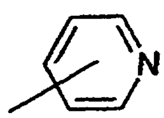
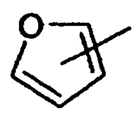
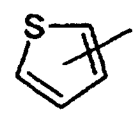
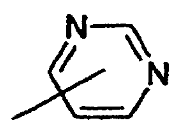

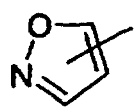
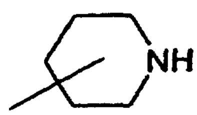
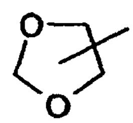
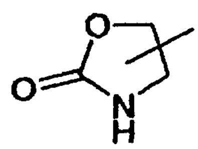

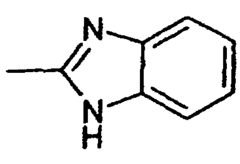
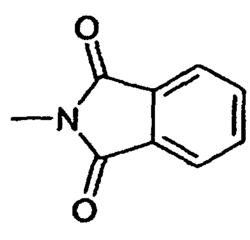
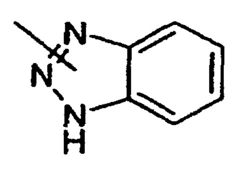

2. Xanthin derivative of the formula (I) as defined in Claim 1 in which $R_1$ represents a pyridin-4-yl group.

3. Xanthin derivative of the formula (I) as defined in either of Claims 1 and 2, whose name is 3,7-dihydro-3-phenyl-1-(4-pyridinylmethyl)1H-purine-2,6-dione hydrochloride.

4. Process for the preparation of compounds of the formula (I) as defined in Claim 1 in a convergent synthesis by coupling two or more fragments which can be derived by retrosynthesis of the compounds of the formula (I).

5. Process according to Claim 4 for the preparation of compounds of the formula (I) as defined in Claim 1, **charac-**

**terized in that** a xanthin of the formula (III),

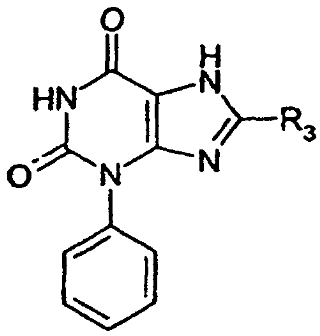

(III)

in which $R_3$ is as defined in Claim 1, undergoes one of the following reactions:

**either**

a) protection of the nitrogen in the 7-position to obtain the compound of the formula (III) in protected form,
b) action of a $R_1-(CH_2)_n-X$ group in the presence of a base, where $R_1$ is defined in claim 1 and n is 1 and X is a halogen or a leaving group, to obtain the intermediate of the formula (IVa),

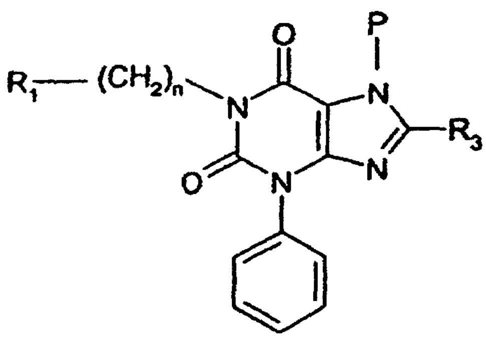

(IVa)

where P is a protecting group,
c) deprotection of the nitrogen in the 7-position,
d) if appropriate, action of an $R_2$-X group in the presence of a base,

**or**

a) action of an $R_2$-X group in the presence of a base to obtain the intermediate of the formula (IVb),

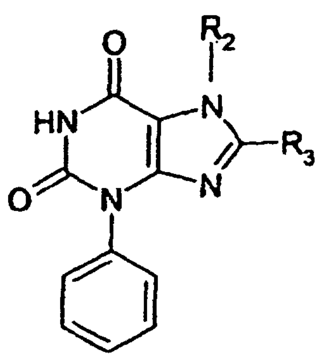

(IVb)

b) action of an $R_1-(CH_2)_n-X$ group in the presence of a base, where $R_1$ and n are as defined above and

X is a halogen or a leaving group, to obtain the compound of the formula (I).

**6.** Compounds of the formula (I) as defined in either of Claims 1 and 2 and/or their pharmaceutically acceptable salts and/or their prodrugs by way of medicament.

**7.** Compound as defined in Claim 3 by way of medicament.

**8.** Medicaments as defined in Claim 6 or 7 intended for the treatment or prevention of osteoporosis.

**9.** Pharmaceutical compositions which comprise, by way of active compound, a medicament as defined in Claims 6 to 8 and one or more excipients.

**10.** Compounds of the formula (IVa) as defined in Claim 5 by way of industrial materials.